# FASCICULE DE BREVET EUROPEEN

(11) **EP 1 177 216 B1**
(45) Date de publication et mention de la délivrance du brevet: **25.08.2004**
(21) Numéro de dépôt: 00922758.8
(22) Date de dépôt: 26.04.2000
(51) Int. Cl.: C08B 30/12, C12P 19/16

(54) **PROCEDE D'OBTENTION DE POLYMERES SOLUBLES DE GLUCOSE BRANCHES**
VERFAHREN ZUR HERSTELLUNG VON VERZWEIGTEN, LÖSLICHEN GLUKOSE-POLYMERISATEN
PROCESS FOR THE MANUFACTURE OF BRANCHED SOLUBLE GLUCOSE POLYMERS

(30) Priorité: 30.04.1999 FR 9905523
(43) Date de publication de la demande: 06.02.2002
(73) Titulaire: Roquette Frères, 62136 Lestrem (FR)
(72) Inventeur: CABOCHE, Jean-Jacques, F-62131 Drouvin Le Marais (FR); LOOTEN, Philippe, F-59130 Lambersat (FR); PETITJEAN, Carole, F-59520 Marquette Lez Lille (FR); FLECHE, Guy, F-59190 Hazebrouck (FR); COMINI, Serge, F-59253 La Gorgue (FR); BACKER, Daniel, F-62350 Saint Venant (FR)
(74) Mandataire: Nargolwalla, Cyra
(86) Numéro de dépôt international: PCT/FR2000/001109
(87) Numéro de publication internationale: WO 2000/066633

(56) Documents cités:
- FR-A- 2 499 588
- CHEMICAL ABSTRACTS, vol. 131, no. 11, 13 septembre 1999 (1999-09-13) Columbus, Ohio, US; abstract no. 143650, KURIKI TAKASHI ET AL.: "Application of cluster dextrin as food ingredient." XP002127630 & NIPPON SHOKUHIN SHINSOZAI KENKYUKAISHI, vol. 1, no. 1, 1998, pages 15-22, jp

## Description

L'invention a pour objet un procédé de fabrication de polymères solubles de glucose branchés ne contenant substantiellement pas de liaisons β glucosidiques, qui présentent des teneurs particulières en liaisons glucosidiques α-1,6, une excellente stabilité en solution exprimée par leur faible tendance à la rétrogradation et une remarquable distribution des poids moléculaires dans un intervalle compris entre 10⁴ et 10⁸ daltons.

Ces polymères solubles de glucose branchés présentent par ailleurs une faible teneur en sucres réducteurs et une faible viscosité.

Au sens de l'invention, les polymères solubles de glucose branchés ne contenant substantiellement pas de liaisons β glucosidiques sont des polymères de glucose lié en α-1,4 et présentant de nombreux points de ramification (encore appelés points de branchement) en α-1,6, et moins de 5 % de branchement en β, c'est-à-dire en β-1,2, β-1,3, β-1,4 ou β-1,6.

Les polymères du glucose classiquement accessibles industriellement sont notamment issus des amidons naturels ou hybrides et de leurs dérivés.

Généralement, l'amidon est constitué de deux polymères, l'amylose et l'amylopectine. L'amylose est la fraction renfermant des homopolymères linéaires de glucose liés en α-1,4 et quelques points de branchement en α-1,6. L'amylopectine est quant à elle la fraction ramifiée, constituée de chaînes linéaires de glucose en α-1,4 reliées à d'autres chaînes linéaires de glucose en α-1,4 par des points de ramification en α-1,6.

L'association de ces deux homopolymères, empaquetés sous la forme de granules d'amidon très bien structurés, constitue la réserve de source carbonée de la plante.

L'amidon produit dans chaque plante est constitué d'un pourcentage variable en chacun de ses constituants amylose et amylopectine, voire même d'une distribution particulière des poids moléculaires de chacun desdits homopolymères de glucose. Ce qui explique la raison pour laquelle les divers amidons et leurs dérivés sont habituellement classés en fonction de leur origine botanique.

Les propriétés fonctionnelles des amidons et de leurs dérivés sont en outre directement fonction de leur contenu en amylose et amylopectine. Ainsi, lorsque l'on chauffe une suspension d'amidon au delà de la température de gélatinisation, le granule d'amidon gonfle, et l'amylose se solubilise préférentiellement. Cependant, lors du refroidissement de la suspension, les homopolymères de glucose rétrogradent, rapidement pour l'amylose (quelques heures), et de manière plus lente pour l'amylopectine (quelques jours).

Or, les spécialistes du domaine de l'utilisation des amidons et des dérivés de l'amidon en industrie alimentaire s'accordent à dire que ce phénomène de rétrogradation affecte la texture des aliments, et en diminue la durée de vie.

Il est connu de rendre plus acceptables ces produits, en les préparant à partir de produits amylacés riches en amylopectine, et donc par exemple à partir des variétés waxy. Cependant, la stabilité des gels et liants obtenus à partir desdits produits amylacés riches en amylopectine n'est pas suffisante pour les besoins des industries alimentaires, où il est parfois nécessaire d'avoir une durée de stockage de plusieurs mois.

Une première solution consiste à stabiliser les homopolymères de glucose, et ce grâce à des agents chimiques. Cette opération est la plupart du temps effectuée par la mise en oeuvre de réactions d'estérification ou d'éthérification. Il peut s'agir notamment de réactions d'acétylation ou d'hydroxypropylation. En outre, pour obtenir les propriétés de texture et de viscosité souhaitées, ces réactions sont souvent associées à une réaction de réticulation.

Ces modifications confèrent alors des propriétés rhéologiques remarquables aux amidons, les rendant plus résistants aux traitements mécaniques tels le cisaillement, ou aux milieux acides. L'acétylation ou l'hydroxypropylation confèrent en outre une bonne stabilité au stockage après cuisson, notamment à basse température.

Cependant, les produits ainsi obtenus présentent l'inconvénient d'avoir été traités chimiquement, ce qui est souvent mal perçu par les consommateurs.

Une seconde solution consiste à isoler l'amidon à partir de plantes dont certains gènes impliqués dans la biosynthèse de l'amidon ont été altérés, ce qui confère aux amidons ainsi modifiés des propriétés particulières.

Il peut s'agir de variétés mutantes ou hybrides, affectées au niveau des gènes waxy (wx), amylose extender (ae), dull (du), opaque (o) shrunken (sh), brittle (bt), ou sugary (su).

Le brevet 4.767.849 décrit ainsi l'amidon extrait d'une variété de maïs homozygote pour le génotype waxy/shrunken-1, qui confère aux amidons granulaires ainsi obtenus des propriétés de stabilité à la rétrogradation en cycles de congélation/décongélation (classiquement appelés cycles de gel/dégel) équivalents aux amidons modifiés chimiquement. Cependant, ces variétés obtenues par croisement entre deux variétés de génotype waxy et shrunken ne présentent qu'une teneur en amidon comprise entre 1 et 20 % de la teneur en amidon normalement synthétisée par les variétés dites de type sauvage.

Il peut s'agir également de plantes génétiquement modifiées, obtenues par modification ciblée d'un gène ou d'un ensemble de gènes codant pour des enzymes intervenant dans la biosynthèse de l'amidon. Les stratégies d'extinction ou d'amplification génique dans la plante, des gènes codant par exemple pour les enzymes de débranchement ou de branchement de l'amidon propres à la plante, ou d'origine exogène, tels les gènes de biosynthèse du glycogène des bactéries, ont été abondamment décrites.

Cependant, force est de constater, comme dans le cas des plantes mutantes ou hybrides, que si les amidons ainsi modifiés présentent des propriétés équivalentes aux amidons modifiés chimiquement, les teneurs en amidon des plantes ainsi obtenues sont loin d'être industriellement satisfaisantes.

Une première alternative à ces procédés consiste à utiliser des enzymes de type α-amylase, α-amylase, pullulanase, iso-amylase pour modifier *in vitro* les amidons natifs afin de leur conférer certaines des propriétés des amidons modifiés chimiquement. Il n'y a donc normalement plus de problèmes liés aux quantités mises en oeuvre.

La demande de brevet EP 539.910 décrit ainsi un procédé de préparation de granules d'amidon modifié par un traitement à l'α-amylase, pour obtenir des produits de moindre viscosité. Cependant, ce procédé ne vise qu'à altérer la structure du granule d'amidon, sans en modifier profondément les constituants.

Le brevet EP 574.721 décrit la préparation d'un produit amylacé à haute teneur en amylopectine stable, en n'utilisant pas de traitement chimique proprement dit, mais en effectuant une réaction d'hydrolyse contrôlée à la β-amylase sur un amidon granulaire natif.

Le produit ainsi préparé présente alors une absence de synérèse et de modification de viscosité dans le temps, et est stable au gel/dégel. Cependant, ce procédé nécessite une étape de traitement thermique préalable, à une température comprise entre 65 et 75°C, pour gélatiniser l'amidon avant de réaliser l'hydrolyse enzymatique proprement dite. De plus, il est surtout nécessaire de contrôler le taux d'hydrolyse pour le limiter à une valeur comprise entre 5 et 20 %.

Une autre alternative aux procédés visant à modifier chimiquement les amidons natifs, ou à extraire des amidons natifs possédant des propriétés d'amidons modifiés à partir de plantes mutantes, hybrides ou génétiquement modifiées, consiste à introduire *in vitro* de nouveaux points de branchement dans l'amidon.

Il s'agit alors de conduire un remaniement des chaînes d'amylopectine ou d'amylose, plutôt que de mettre en oeuvre des réactions de stabilisation et/ou de réticulation comme indiqué précédemment.

Deux techniques sont habituellement mises en oeuvre. La première utilise des moyens thermiques, la seconde des enzymes purifiées de biosynthèse du glycogène et/ou de l'amidon, telles les enzymes de branchement du glycogène ou de l'amidon, responsables respectivement de la synthèse des points de ramification en α-1,6 du glycogène, ou des points de ramification en α-1,6 de l'amylopectine et des quelques points de branchement de l'amylose.

La demande de brevet WO 95/22562 décrit par exemple des dextrines de type amidon, caractérisées par leur poids moléculaire compris entre 15.10³ et 10⁷ daltons, et un degré de branchement compris entre 2 et 8 %, obtenues par le traitement, en conditions acides (acide orthophosphorique à 0,17 % en poids d'amidon) et à une température comprise entre 110 à 140°C pendant de 1 à 15 h, d'amidon granulaire natif, notamment de la fécule de pomme de terre.

La composition ainsi obtenue est destinée aux sportifs comme apport énergétique après un effort physique. Cependant, ce traitement est long et très lourd à mettre en oeuvre, et il conduit à des polymères de glucose qui renferment, outre une teneur élevée en liaisons α-1,6 (de préférence comprise entre 3 et 7 %), de nouveaux types de liaisons qui n'existent pas normalement dans l'amidon natif. Les analyses par résonance magnétique nucléaire (RMN) révèlent en effet des liaisons de type β-1,4, β-1,6, et des liaisons α autres qu'en α-1,4 et en α-1,6.

Le document FR-A-2.499.588 (respectivement la publication Nippon Shokuhin Shinsozai Kenk. (1998), 1/1, p. 15-22) décrit l'obtention de polymères de glucose (resp. de mégacycles de dextrines), par réaction d'une enzyme branchant sur une substance amylacée. Ces polymères sont différents des composés de la présente invention.

De tout ce qui précède, il résulte qu'il y a donc un besoin non satisfait de disposer, d'une part, de polymères de glucose présentant des propriétés remarquables, notamment en terme de stabilité, de solubilité et éventuellement de viscosité et conférant par la même aux produits qui les contiennent des capacités plus grandes de durées de vie et de digestibilité, et d'autre part, de les obtenir sans utiliser de techniques chimiques ou physiques, ni d'avoir recours à des extractions à partir de plantes mutantes ou génétiquement modifiées.

La société Demanderesse a eu le mérite de concilier tous ces objectifs réputés jusqu'alors difficilement conciliables en imaginant et élaborant, au prix de nombreuses recherches, un procédé de fabrication de polymères solubles de glucose branchés ne contenant substantiellement pas de liaisons β glucosidiques.

Les polymères solubles de glucose branchés ne contenant substantiellement pas de liaisons β glucosidiques obtenus au moyen du procédé conforme à l'invention sont ainsi caractérisés par le fait qu'ils possèdent entre 2,5 et 10 % de liaisons glucosidiques α-1,6, une tendance très faible ou nulle à la rétrogradation en solution aqueuse, déterminée selon un test A et un Mp déterminé selon un test C à une valeur centrée du profil de distribution des masses moléculaires comprise entre 10⁴ et 10⁸ daltons.

Les polymères de glucose branchés conformes à l'invention présentent par ailleurs une faible teneur en sucres réducteurs, au plus égale à 9 % et une viscosité déterminée selon un test B, pour 3 g de produit sec, au plus égale à 5.000 cP (5.000 m Pa s).

La teneur en liaisons glucosidiques α-1,6 des polymères solubles de glucose branchés conformes à l'invention, déterminée par analyse RMN du proton, est de 2,5 à 10 %, exprimée en nombre de liaisons α-1,6 par rapport au nombre total de liaisons glucosidiques α-1,4 et α-1,6 des dits polymères de glucose branchés.

Cette teneur en liaisons glucosidiques α-1,6, confère à tout polymère de glucose conforme à l'invention une structure particulière, en termes de degré de ramification et/ou de longueurs de chaînes ramifiées en regard de l'amidon ou du dérivé d'amidon dont il est issu.

Les polymères solubles de glucose branchés conformes à l'invention présentent également une faible tendance à la rétrogradation en solution aqueuse, déterminée selon un test A. Ce test consiste à établir l'aptitude à la rétrogradation d'un produit donné au cours de cycles répétés de gel/dégel.

La rétrogradation observée du produit, et l'enthalpie de déstructuration du produit qui a pu rétrograder, déterminée par Analyse Calorimétrique Différentielle, renseignent donc sur la stabilité du produit considéré.

Le test A consiste plus précisément à effectuer une préparation aqueuse du produit à tester à 40 % de matière sèche. On effectue différents prélèvements dans des creusets hermétiquement clos. L'ensemble des creusets est porté pendant 15 min à une température de 100°C pour réaliser la gélatinisation ou la mise en solution, et on soumet ensuite ces creusets à un traitement de cycles de gel/dégel, chacun des cycles consistant à amener et maintenir ladite préparation pendant 15 min à une température de -20°C, puis à une température de 20°C et à la maintenir ensuite pendant 1 h 30 à cette température.

Une analyse calorimétrique différentielle est ensuite réalisée à chaque cycle, sur équipement PERKIN ELMER, pour la détermination de l'enthalpie de déstructuration du produit qui a pu alors rétrograder.

La stabilité aux cycles de gel/dégel s'apprécie donc en premier lieu par le nombre de cycle de gel/dégel au delà duquel on peut réaliser cette mesure de la valeur d'enthalpie requise pour déstructurer le gel d'amidon qui a alors rétrogradé.

Les polymères de glucose obtenus au moyen du procédé conforme à l'invention soumis à ces cycles répétés de gel/dégel présentent, de manière surprenante et inattendue, une « faible tendance à la rétrogradation », c'est-à-dire ici une absence partielle, voire totale de rétrogradation selon le test A et en fonction de leur teneur en liaisons glucosidiques α-1,6.

C'est ainsi que les polymères de glucose obtenus au moyen du procédé conforme à l'invention qui présentent une teneur en liaisons glucosidiques α-1,6 comprise entre 2,5 et 5 %, ne commencent à rétrograder significativement qu'au delà du huitième cycle de gel/dégel, en présentant une faible valeur d'enthalpie de rétrogradation, comme il sera exemplifié ci-après.

On les qualifie de polymères de glucose branchés présentant une « très faible tendance à la rétrogradation».

Quant aux polymères de glucose obtenus au moyen du procédé conforme à l'invention qui présentent une teneur en liaisons glucosidiques α-1,6 comprise entre 5 et 10 %, aucune rétrogradation de la solution n'est constatée même après 12 cycles de gel/dégel, ce qui explique pourquoi aucune enthalpie de déstructuration ne peut être établie.

Il est particulièrement surprenant que les polymères de glucose obtenus au moyen du procédé conforme à l'invention, puissent présenter une telle stabilité. En effet, les mesures effectuées avec le test A, sur les amidons waxy et les amidons waxy réticulés. et acétylés, (tels ceux préparés en suivant les enseignements du brevet US 2.928.828) rétrogradent entre le quatrième et le sixième cycle de gel/dégel, comme il sera montré dans l'exemple 2.

Il n'existe donc pas, à la connaissance de la société Demanderesse, de polymères de glucose qui présentent une telle stabilité.

Cette propriété destine tout naturellement les polymères de glucose branchés obtenus au moyen du procédé conforme à l'invention à des compositions utilisables en industrie alimentaire, qui présentent alors des stabilités élevées au stockage.

Un autre avantage de l'invention est de permettre l'obtention d'un produit fini, utilisable par exemple comme liant instantané dans des produits réfrigérés ou surgelés.

la détermination de la valeur centrée du profil de distribution des masses moléculaires des polymères solubles de glucose branchés obtenus au moyen du procédé conforme à l'invention est réalisée par la mesure de la distribution des masses moléculaires moyennes en poids (Mp).

Dans la pratique les valeurs de Mp ne se calculent pas, mais se mesurent par différentes techniques. On utilise par exemple une méthode de mesure adaptée aux polymères de glucose, qui repose sur la chromatographie de perméation de gel sur des colonnes de chromatographie étalonnées avec des pullulans de masses moléculaires connues.

Le test C, mis au point par la société Demanderesse pour déterminer la valeur centrée du profil de distribution des masses moléculaires caractéristiques des polymères solubles de glucose branchés obtenus au moyen du procédé conforme à l'invention, consiste :
- à établir le profil de distribution molaire des fractions chromatographiques desdits polymères solubles de glucose branchés,
- à déterminer la valeur dite « valeur centrée du profil de distribution des masses moléculaires » qui correspond à la valeur du pic moyen de distribution des poids moléculaires de la population représentant plus de 90% des fractions chromatographiques issues de ladite chromatographie séparative de perméation de gel.

Les polymères de glucose branchés obtenus au moyen du procédé conforme à l'invention présentent alors une valeur Mp centrée du profil de distribution de masses moléculaires comprise entre 10⁴ et 10⁹ daltons.

De manière avantageuse, les polymères solubles de glucose obtenus au moyen du procédé conforme à l'invention peuvent être classés en deux familles, la première famille présentant une valeur de Mp centrée du profil de distribution de masses moléculaires comprise entre 10⁵ et 10⁶ daltons, et la seconde famille présente une valeur de Mp centrée du profil de distribution de masses moléculaires compris entre 10⁷ et 10⁸ daltons.

Les polymères solubles de glucose branchés obtenus au moyen du procédé conforme à l'invention présentent par ailleurs une faible teneur en sucres réducteurs.

La détermination du pouvoir réducteur des polymères de glucose branchés obtenus au moyen du procédé conforme à l'invention, par toute méthode connue par ailleurs de l'homme du métier, conduit à des valeurs au plus égale à 9 %.

De manière avantageuse, les polymères de glucose branchées peuvent être classés en deux sous-familles en fonction de leur teneur en sucres réducteurs.

La première sous-famille présente une teneur en sucres réducteurs au plus égale à 1 %.

La seconde sous-famille présente une teneur en sucres réducteurs comprise entre 5,5 et au plus 9 %.

La société Demanderesse a en outre trouvé que les polymères de glucose branchés obtenus au moyen du procédé conforme à l'invention présentent des profils rhéologiques tout à fait particuliers.

L'analyse de viscosité des polymères de glucose branchés obtenus au moyen du procédé conforme à l'invention est réalisée grâce à un test B mis au point par la société Demanderesse pour cette gamme particulière de produits.

Il ne s'agit pas en effet ici de produits granulaires tels qu'habituellement décrits et analysés dans l'état de la technique, mais de polymères de glucose branchés qui présentent de manière surprenante et inattendue une remarquable solubilité dans l'eau froide.

Le test B consiste à préparer tout d'abord le produit à analyser par précipitation à l'éthanol, séchage sous vide puis broyage au mortier, et enfin tamisage sur tamis de maille 125 µm. Une masse comprise entre 3 et 15 g du produit sec à analyser ainsi obtenu est alors introduite, avec 6,75 g de glycérol à 98 % de pureté, dans le bol d'un Rapid Visco Analyser (RVA - NewPort Scientific), et l'ensemble est soigneusement homogénéisé à l'aide d'une micro-spatule.

Une quantité d'eau déminéralisée est ensuite ajoutée, afin d'obtenir une masse finale de 28 g. L'ensemble est alors immédiatement agité. Le profil d'analyse temps / température et vitesse dans le RVA est alors réalisé comme suit. L'échantillon est agité à 100 rpm à une température de 25°C durant 5 s, puis à 500 rpm pendant 25 s. L'agitation est alors maintenue à 160 rpm durant le reste du profil. La température initiale de 25°C est maintenue durant 10 min, puis elle est augmentée à 90°C en 8 min. Cette température de 90°C est ensuite maintenue 3 min, diminuée à 30°C en 8 min, puis maintenue à cette valeur de 30°C durant 5 min.

La viscosité retenue est la viscosité mesurée en centipoises (cP) (m Pa.s) en fin de profil d'analyse, à 34 min.

Les polymères de glucose branchés obtenus au moyen du procédé conforme à l'invention présentent alors une viscosité au plus égale à 5.000 cP (5000 m Pa s) pour 3 g sec de produit.

La société Demanderesse a également trouvé que ces valeurs de viscosité des polymères de glucose branchés obtenus au moyen du procédé conforme à l'invention sont du même ordre de grandeur que les valeurs de viscosité, déterminées en suivant le même test B, des amidons waxy fluidifiés par traitement acide.

Cependant, des analyses complémentaires de mesure de viscosité effectuées après sept jours de stockage à 4°C, ont permis de mettre en évidence, de manière surprenante et inattendue, une remarquable stabilité de la viscosité des polymères de glucose branchés, contrairement auxdits amidons waxy fluidifiés de même viscosité, comme il sera exemplifié ci-après.

Ces produits peuvent donc être par exemple avantageusement utilisés pour la fabrication de préparations alimentaires liquides instantanées, et surtout peuvent permettre d'assurer le stockage de longue durée à basse température.

Les polymères solubles de glucose branchés obtenus au moyen du procédé conforme à l'invention sont donc particulièrement bien adaptés à des compositions destinées à être utilisées dans les industries notamment du Papier-Carton, du Textile, de la Pharmacie, de la Cosmétique, et ainsi en particulier de l'Alimentaire.

Pour préparer ces polymères solubles de glucose branchés, on réalise la succession des étapes suivantes qui consiste en ce que l'on:
a) soumet une suspension aqueuse d'amidon ou de dérivé d'amidon d'une matière sèche au moins égale à 1 % en poids, de préférence de 2 à 50 % en poids, à une température supérieure à 130°C, de préférence comprise entre 140 et 150°C, sous une pression de plus de 3,5 bars, de préférence comprise entre 4 à 5 bars pendant au moins 2 minutes, de préférence pendant 2 à 5 minutes,
b) traite l'amidon ainsi obtenu avec 50 à 2.000 unités d'enzyme de branchement purifiée à une température comprise entre 25 et 50°C, de préférence à une température de 30°C pendant une durée de 10 min à 24 heures,
c) recueille les polymères de glucose branchés ainsi obtenus.

L'amidon est introduit en solution aqueuse à une matière sèche au moins égale à 1 % en poids, de préférence de 2 à 50 % en poids.

Le choix d'une origine, ou d'une qualité d'amidon ou de ses dérivés particuliers, ne revêt qu'une importance relative.

La société Demanderesse a trouvé que les polymères de glucose branchés conformes à l'invention sont aisément synthétisables à partir d'amidons ou de leurs dérivés, qui présentent déjà un taux de branchement au moins égal à 1 %.

Cette suspension d'amidon ou de dérivés d'amidon est soumise ensuite à un traitement par cuisson particulier, qui consiste à la traiter à une température supérieure à 130°C, de préférence comprise entre 140 et 150°C, sous une pression de plus de 3,5 bars, de préférence comprise entre 4 à 5 bars pendant au moins 2 minutes, de préférence pendant 2 à 5 minutes. Ce traitement est réalisé avantageusement dans un cuiseur tubulaire à double enveloppe chauffé par fluide thermique, équipement qu'il est aisé à l'homme du métier de se procurer.

La deuxième étape du procédé conforme à l'invention consiste à traiter l'amidon ainsi obtenu avec de 50 à 2.000 unités d'enzyme de branchement purifiée à une température comprise entre 25 et 50°C, de préférence à une température de 30°C pendant une durée de 10 min à 24 heures.

Les enzymes de branchement sont choisies dans le groupe constitué des enzymes de branchement du glycogène et des enzymes de branchement de l'amidon et les mélanges quelconques de ces enzymes. Plus préférentiellement, on choisit l'enzyme de branchement du glycogène de *Escherichia coli*, et les enzymes de branchement de l'amidon, et plus préférentiellement encore les enzymes de branchement de l'amidon de type I et de type II du maïs, ou encore de l'amidon d'algue unicellulaire, par exemple celles de l'algue verte *Chlamydomonas reinhardtii*.

L'isolement des dites enzymes de branchement du glycogène ou de l'amidon peut être effectué par tout moyen connu en soi par l'homme du métier.

Concernant les enzymes de branchement d'algue unicellulaire, la société Demanderesse recommande cependant de mettre en oeuvre le procédé de préparation décrit dans la demande de brevet français déposée sous le n° 98/12051, dont elle est titulaire.

L'accès aux enzymes purifiées peut être réalisé à partir du mélange d'enzymes d'algue ainsi obtenu, en mettant en oeuvre directement des techniques de séparation chromatographique en elles-mêmes connues, ou par l'utilisation des techniques de l'ADN recombinant.

Il peut être en effet avantageux de préférer isoler et exprimer les gènes codant pour les enzymes de branchement de l'amidon d'algue unicellulaire dans un micro-organisme plus facilement manipulable que les algues unicellulaires.

La technique, connue en soi par l'homme du métier, consiste alors par exemple à :
- produire des anticorps polyclonaux spécifiques de chacune des enzymes de branchement de l'amidon d'algue préalablement purifiée,
- cribler, avec lesdits anticorps spécifiques, une banque d'expression d'ADN génomique de l'algue unicellulaire considérée,
- isoler les fragments d'ADN à partir des clones de ladite banque d'expression d'ADN génomique qui ont réagi avec l'un et/ou l'autre des anticorps polyclonaux spécifiques,
- introduire lesdits fragments d'ADN correspondants aux gènes codant pour les enzymes de branchement de l'amidon d'algue unicellulaire dans des bactéries permettant leur expression.

Les enzymes de branchement de l'amidon d'algue produites par ce procédé sont dites des enzymes de branchement recombinantes, puisque en provenance d'une algue unicellulaire, puis transférées génétiquement et exprimées dans un micro-organisme d'une autre espèce, en l'occurrence ici une bactérie.

Pour préparer les polymères solubles de glucose branchés conformes à l'invention, on peut donc faire agir avantageusement une enzyme de branchement de l'amidon d'algue purifiée recombinante sur une colle d'amidon de maïs waxy préparé selon l'étape a) dudit procédé.

La dernière étape du procédé conforme à l'invention consiste donc à collecter les polymères de glucose branchés ainsi obtenus.

Les produits sont précipités par 3 volumes d'éthanol, purifiés et séchés sous vide pendant 24 heures, ou encore atomisés, par toute technique connue par ailleurs de l'homme du métier.

D'autres caractéristiques et avantages de l'invention apparaîtront à la lecture des exemples non limitatifs décrits ci-dessous

### EXEMPLE 1

La préparation des polymères de glucose branchés s'effectue comme suit. On prépare une suspension d'amidon de maïs waxy d'une teneur en matière sèche de 2,5 % en poids. On traite ensuite cette suspension dans un cuiseur tubulaire à double enveloppe chauffé par fluide thermique de laboratoire, à une température de 145°C, sous une pression de 4 bars. Le débit d'alimentation est de 40 ml/min, pour un temps de séjour dans ledit cuiseur de 3 minutes.

1,5 litres de cette préparation sont refroidis à température ambiante et placés dans un milieu tamponné à pH 7 par du tampon Tris HCl 0,1 M final pour un volume total de 3,750 litres. On ajoute 19 ml (d'une solution enzymatique à 1,8 mg/ml de protéines, présentant en outre une activité spécifique de 1.100 U/mg, activité mesurée par la méthode de dosage à la phosphorylase A connue en soi par l'homme du métier) d'une solution d'enzymes de branchement de l'amidon recombinantes de l'algue *Chlamydomonas reinhardtii* préalablement purifiées, et on laisse agir à 30°C pendant 30 min pour obtenir des polymères de glucose branchés conformes à l'invention présentant une teneur en liaisons glucosidiques α-1,6 de 4,3 % (produit A), et pendant 2 heures, pour obtenir des polymères de glucose branchés, conformes à l'invention, présentant une teneur en liaisons glucosidiques α-1,6 de 6 % (produit B). Chacun des produits est ensuite précipité à l'éthanol, filtré, rincé et séché sous vide pendant 24 heures.

Les valeurs respectives des Mp centrées du profil de distribution des masses moléculaires des produits A et B sont respectivement de 1,5.10⁷ daltons et de 2,2.10⁷ daltons. Leurs teneurs en sucres réducteurs est respectivement de 0,05 % et de 0,07 %.

### EXEMPLE 2

La détermination de la stabilité des polymères de glucose branchés obtenus au moyen du procédé conforme à l'invention est réalisée par la mesure de l'enthalpie de déstructuration du produit rétrogradé, si produit rétrogradé il y a, par Analyse Calorimétrique Différentielle, au cours de cycles répétés de gel/dégel.

Deux polymères de glucose branchés conformes à l'invention, présentant respectivement une teneur en liaisons glucosidiques α-1,6 de l'ordre de 4,3 % (produit A) et de l'ordre de 6 % (produit B) sont préparés comme indiqué dans l'exemple 1. L'analyse est également effectuée sur deux autres échantillons : de l'amidon de maïs waxy (produit C) et un amidon waxy réticulé et acétylé, présentant un indice d'acétyle de 1,8 (produit D).

Comme indiqué dans le test A, on constitue une préparation aqueuse de chacun des 4 échantillons à 40 % de matière sèche placés dans un ensemble de creusets hermétiquement clos, et on chauffe pendant 15 min à 100°C dans un four DSC4 de PERKIN ELMER. On réalise pour chaque creuset de 2, 4, 6, 8, 10 ou 12 cycles successifs de gel/dégel en suivant le protocole suivant : 15 min à -22°C, puis 1h30 à 20°C. Une mesure de l'enthalpie de rétrogradation est effectuée sur chaque creuset en le plaçant dans le calorimètre différentiel PERKIN ELMER.

Le tableau I suivant présente les mesures d'enthalpie de rétrogradation déterminées pour chacun des 4 produits testés au cours de 12 cycles successifs de gel/dégel.

**Tableau I.**

| Détermination des enthalpies de rétrogradation au cours des 12 cycles de gel/dégel, exprimées en J/g de préparation. | | | | | |
|---|---|---|---|---|---|
| **PRODUITS** | **Cycle 2** | **Cycle 4** | **Cycle 6** | **Cycle 8** | **Cycle 12** |
| A | 0 | 0 | 0 | 0 | 0,2 |
| B | 0 | 0 | 0 | 0 | 0 |
| C | 0 | 0 | 0,4 | 1 | 2,2 |
| D | 0 | 0,10 | 0,35 | 0,6 | 1,75 |

Les polymères de glucose branchés présentent donc une remarquable stabilité, même après 12 cycles de gel/dégel. Si l'amidon waxy (produit C) et l'amidon waxy réticulé et acétylé (produit D) commencent à rétrograder à partir du 4^{ème} cycle de gel/dégel, il n'en est pas de même pour chacun des polymères de glucose branchés conformes à l'invention préparés à partir dudit amidon waxy. Le procédé enzymatique mis en oeuvre pour modifier les amidons et dérivés d'amidon permet donc de leur assurer une excellente stabilité, bien supérieure en l'état aux amidons waxy stabilisés et/ou réticulés.

### EXEMPLE 3

La caractérisation rhéologique des polymères de glucose branchés est réalisée à l'aide d'un Rapid visco Analyser (RVA).

Les produits conformes à l'invention présentant une remarquable solubilité dans l'eau froide.

Il a donc été nécessaire de mettre au point une méthode de détermination de viscosité propre à ce type de produit.

Comme indiqué dans le test B, 4,5 g du produit sec à tester sont mélangés avec du glycérol et de l'eau pour atteindre une masse finale de 28 g.
Les produits analysés sont d'une part, les produits A, B et C décrits dans l'exemple 2 et deux autres produits E et F, correspondants à des amidons de maïs waxy fluidifiés à deux niveaux de fluidification (valeur appréciée par la mesure classique de la fluidité dans l'eau, i.e. l'indice de " water fluidity " ou WF), obtenus par traitement en conditions acides connues en soi par l'homme du métier, le produit E présentant un WF de 50, et le produit F, un WF de 65.

Le profil d'analyse temps / température et vitesse dans le RVA est alors réalisé comme suit. L'échantillon est agité à 100 rpm à une température de 25°C durant 5 s, puis à 500 rpm pendant 25 s. L'agitation est alors maintenue à 160 rpm durant le reste du profil.

La température initiale de 25 °C est maintenue durant 10 min, puis elle est augmentée à 90°C en 8 min.

Cette température de 90°C est ensuite maintenue 3 min, diminuée à 30°C en 8 min, puis maintenue à cette valeur de 30°C durant 5 min.

Le tableau II suivant présente les résultats de viscosité pour les produits A, B, C, E et F, exprimés en centipoises (= m Pa.s).

**Tableau II.**

| Détermination des viscosités en fin de profil temps/température et vitesse en RVA des produits A, B, C, E et F, exprimées en centi-Poises (cP) (= m Pa.s). | |
|---|---|
| **PRODUITS** | **Viscosité à 34 min** |
| A | 1600 |
| B | 750 |
| C | 6060 |
| E | 1140 |
| F | 660 |

Les polymères de glucose branchés obtenus au moyen du procédé conforme à l'invention présentent encore une certaine viscosité, mais remarquablement plus faible que celle du témoin amidon waxy (C).

On remarque que ces valeurs de viscosité sont du même ordre de grandeur que les amidons waxy fluidifiés.

Une étude complémentaire est réalisée par mesure de la viscosité après stockage durant 7 jours à 4°C.

Cette étude permet de caractériser la stabilité des colles ainsi fabriquées dans le temps et de déterminer en quoi les polymères de glucose branchés obtenus au moyen du procédé conforme à l'invention diffèrent des amidons waxy fluidifiés.

On réalise le stockage des bols du RVA contenant chacun des cinq produits à 4°C.

La viscosité est ensuite à nouveau déterminée par le RVA. Le profil d'analyse temps / température et vitesse est alors caractérisé par une vitesse et une température maintenues respectivement à 160 et 30°C pendant 20 min.

La viscosité retenue est la viscosité moyenne mesurée en cP (=m Pa.s) entre 15 et 20 min.

Le tableau III suivant présente les résultats de viscosité obtenus après 7 jours de stockage à 4°C des produits A, B, C, E et F.

**Tableau III.**

| Détermination des viscosités des produits après stockage pendant 7 jours à 4°C, exprimées en cP (= m Pa.s). | |
|---|---|
| **PRODUITS** | **Viscosité après** **7 jours à 4 °C** |
| A | 2500 |
| B | 850 |
| C | 8650 |
| E | Gel blanc, dur, ferme* |
| F | Cel blanc, dur, ferme* |

| | |
|---|---|
| * : viscosité non mesurable. | |

Les résultats montrent clairement que les polymères de glucose branchés obtenus au moyen du procédé conforme à l'invention présentent une viscosité remarquablement stable même après un stockage à 4°C. Cette faible viscosité peut donc être mise à profit avantageusement pour des préparations alimentaires qui nécessitent que l'ingrédient amylacé qui les composent soit de faible viscosité (telles que les préparations liquides instantanées) et qui demandent à être stockées pendant une longue période de temps à basses températures.

### EXEMPLE 4

On prépare des polymères solubles de glucose branchés obtenus au moyen du procédé conforme à l'invention, en faisant agir une enzyme de branchement du glycogène isolée de *E. coli* sur divers solutions d'amidons et dérivés d'amidon, pendant 21 heures. de réaction à 30°C et conformément aux autres conditions décrites dans l'exemple 1.

Il s'agit en l'occurrence ici de suspensions d'amidon standard de maïs (G), d'amidon de maïs waxy (I), d'amidon riche en amylose commercialisé par la société Demanderesse sous le nom d'EURYLON® 7 (K) et d'une maltodextrine commercialisée par la société Demanderesse sous le nom de GLUCIDEX® 2 (M).

Le tableau IV suivant présente les résultats obtenus en terme de teneurs en liaisons glucosidiques α-1,6, de valeur des Mp centrées du profil de distribution des poids moléculaires, de teneur en sucres réducteurs et de comportement de rétrogradation après 10 cycles de gel/dégel.

**Tableau IV.**

| Détermination des caractéristiques physico-chimiques et fonctionnelles des polymères solubles de glucose obtenus au moyen du procédé conforme à l'invention H, J, L et N obtenus par l'action de l'enzyme de branchement du glycogène de *E. coli* respectivement sur les substrats G, I, K et M à une matière sèche donnée. | | | | | | | | |
|---|---|---|---|---|---|---|---|---|
| | **G** **10% MS** | **H** | **I** **1% MS** | **J** | **K** **5 % MS** | **L** | **M** **20% MS** | **N** |
| Teneur en liaisons glucosidiques α-1,6 (%) | 3 | **3,4** | 4,4 | **5,6** | 1,9 | **3,3** | 6,1 | **7,1** |
| Valeur de Mp centrée (daltons) | 5.10⁷ | **5,8.10**^{**5**} | 1.10⁸ | **2,2.10**^{**5**} | 8,5.10⁶ | **5.10**^{**5**} | 3,3.10⁵ | **1,4.10**^{**5**} |
| Teneur en sucres réducteurs | 0,13 | **0,16** | < 0,5 | **< 0,05** | 0,5 | **0,5** | 3 | **3,5** |
| Enthalpies de rétrogradation (J/g) | 2 | **1** | 1,5 | **0** | 3 | **0,4** | 2,3 | **0** |

Les polymères solubles de glucoses branchés obtenus au moyen du procédé conforme à l'invention présentent donc une remarquable tenue au gel/dégel et une distribution des poids moléculaires centrés sur un fin intervalle de valeurs compris entre 1,4 et 5,8. 10⁵ daltons, alors que les substrats de départ présentent au contraire une forte tendance à la rétrogradation et des profils de distribution des poids moléculaires étalés de 10³ à 10⁸ daltons.

## Revendications

1. Procédé de fabrication de polymères de glucose branchés ne contenant substantiellement pas de liaisons β glucosidiques, c'est-à-dire contenant moins de 5 % de branchements en β, à partir d'une suspension aqueuse d'amidon ou de dérivé d'amidon d'une matière sèche au moins égale à 1% en poids, de préférence de 2 à 50 % en poids, **caractérisé par le fait que l'on :**
- soumet ladite suspension d'amidon ou de dérivé d'amidon à une température supérieure à 130°C, de préférence comprise entre 140 et 150°C, sous une pression de plus de 3,5 bars, de préférence comprise entre 4 et 5 bars, pendant au moins 2 minutes, de préférence pendant 2 à 5 minutes,
- traite l'amidon ou le dérivé d'amidon ainsi obtenu avec de 50 à 2.000 unités d'une enzyme de branchement purifiée, l'enzyme de branchement étant extraite d'organismes et/ou de microorganismes choisis dans le groupe constitué par les plantes supérieures, les levures, les bactéries et les algues unicellulaires, et est de préférence extraite d'algues unicellulaires, à une température comprise entre 25 et 50°C , de préférence à une température de 30°C, pendant une durée de 10 minutes à 24 heures, et
- recueille les polymères de glucose branchés ainsi obtenus.

2. Procédé de fabrication de polymères solubles de glucose branchés selon la revendication 1 tel que l'enzyme de branchement est choisie dans le groupe formé par les enzymes de branchement du glycogène de *Escherichia coli,* et les enzymes de branchement de l'amidon d'algue unicellulaire, par exemple celles de l'algue verte *Chlamydomonas reinhardtii.*

3. Procédé de fabrication de polymères solubles de glucose branchés selon l'une ou l'autre des revendications 1 ou 2, tel que l'enzyme de branchement est extraite d'algues et obtenue par isolement à partir d'un organisme génétiquement modifié capable d'exprimer ladite enzyme.

## Patentansprüche

1. Verfahren zur Herstellung von verzweigten Glukosepolymeren, die im wesentlichen keine β-glucosidischen Bindungen enthalten, d. h. sie enthalten weniger als 5 % β-Verzweigungen, aus einer wässerigen Suspension von Stärke oder einem Stärkederivat mit einer Trockenmasse von wenigstens gleich 1 Gew.-%, vorzugsweise von 2 bis 50 Gew.-%, **dadurch gekennzeichnet, dass**
- die Suspension von Stärke oder einem Stärkederivat einer Temperatur größer als 130 °C, vorzugsweise umfasst zwischen 140 und 150°C, unter einem Druck von mehr als 3,5 bar, vorzugsweise umfasst zwischen 4 und 5 bar, für wenigstens 2 Minuten, vorzugsweise für 2 bis 5 Minuten, unterzogen wird,
- die auf diese Weise erhaltene Stärke oder das auf diese Weise erhaltene Stärkederivat mit 50 bis 2.000 Einheiten eines gereinigten Verzweigungsenzyms, wobei das Verzweigungsenzym aus Organismen und/oder Mikroorganismen extrahiert wird, die aus der Gruppe gebildet aus höheren Pflanzen, Hefen, Bakterien und einzelligen Algen ausgewählt werden, und vorzugsweise aus einzelligen Algen extrahiert wird, bei einer Temperatur umfasst zwischen 25 und 50°C, vorzugsweise bei einer Temperatur von 30 °C für eine Dauer von 10 Minuten bis 24 Stunden behandelt wird,
- die verzweigten Glucosepolymere, die auf diese Weise erhalten wurden, gesammelt werden.

2. Verfahren zur Herstellung von löslichen verzweigten Glucosepolymeren nach Anspruch 1, **derartig, dass** das Verzweigungsenzym aus der Gruppe gebildet aus den Verzweigungsenzymen von Glykogen aus *Escherichia coli* und den Verzweigungsenzymen von Stärke aus einzelligen Algen, z. B. von solchen der Grünalge *Chlamydomonas reinhardtii*, ausgewählt wird.

3. Verfahren zur Herstellung von löslichen verzweigten Glucosepolymeren nach einem der Ansprüche 1 oder 2, derartig, dass das Verzweigungsenzym aus Algen extrahiert wird und durch Isolieren aus einem genetisch veränderten Organismus erhalten wird, der fähig ist, das Enzym zu exprimieren.

## Claims

1. Process for manufacture of branched polymers of glucose essentially containing no β-glucosidic bonds, that is to say containing less than 5% of β-branching, from an aqueous solution of starch or of starch derivative of dry matter of at least 1% by weight, preferably 2 to 50% by weight, **characterized by** the fact that :
- such a suspension of starch or of starch derivative is subjected to a temperature greater than 130°C, preferably lying between 140 and 150°C, under a pressure of more than 3.5 bars, preferably lying between 4 and 5 bars, for at least 2 mins, preferably for 2 to 5 mins,
- the starch or starch derivative thus obtained is treated with 50 to 2,000 units of purified branching enzyme at the branching enzyme, the branching enzyme being extracted from organisms and/or from microorganisms selected from the group consisting of higher plants, yeasts, bacteria and unicellular algae, and being preferably extracted from unicellular algae at a temperature lying between 25 and 50°C, preferably at a temperature of 30°C, for a duration from 10 mins to 24 hrs, and
- the branched polymers of glucose thus obtained are collected.

2. Process for manufacture of soluble branched polymers of glucose according to Claim 1, wherein the branching enzyme is selected from the group consisting in glycogen branching enzymes of *Escherichia Coli,* and the branching enzymes of unicellular algal starch, for example these of the green algae *Chlamydomonas reinhardtii.*

3. Process for manufacture of soluble branched polymers of glucose according to one of the claims 1 or 2, wherein the branching enzyme extracted from algae is obtained by isolation from a genetically modified organism capable of expressing the said enzyme.
